# EUROPEAN PATENT APPLICATION

(11) **EP 1 493 414 A1**
(43) Date of publication of application: **05.01.2005**
(21) Application number: 04253898.3
(22) Date of filing: 29.06.2004
(51) Int. Cl.: A61F 13/15, A61L 15/62

(54) **Disposable absorbent article**

(30) Priority: 30.06.2003 US 609740
(71) Applicant: McNeill-PPC, Inc., Skillman, NJ 08858 (US)
(72) Inventor: Bhupendra, Vipul, Hillsborough New Jersey 08844 (US); Haywood, Dexter L., Franklin Park New Jersey 08823 (US); Joseph, Annemarie Devine, Plainsboro New Jersey 08536 (US)
(74) Representative: Mercer, Christopher Paul

(57) **Abstract**

A biodegradable, water dispersible absorbent article is disclosed. The absorbent article includes an absorbent core, a water insoluble, biodegradable film, and a water dispersible outer shell;
wherein the water insoluble, biodegradable film is laminated to the absorbent core.

## Description

### Field of the Invention

The invention relates to absorbent articles for use with undergarments or other clothing, such as, panty hose, swimsuits, or leotards. In particular, the absorbent articles of the present invention may be disposed in a standard toilet system.

### Background of the Invention

Disposable absorbent articles, such as, pantiliners, sanitary napkins, interlabial devices, adult incontinence devices, bandages, and diapers are well known in the art. These articles typically have a fluid permeable body-facing side and fluid impermeable garment facing side. Additionally, such articles may include an absorbent layer for retaining fluids therebetween.

Typical disposal methods of such articles are generally considered to be unsatisfactory as the articles usually end up being incinerated or in a landfill. An alternate disposal method involves flushing the article down the toilet and subsequently degrading it in the municipal or private sewage or septic system. This disposal method is generally considered convenient and discrete. For this disposal method, the suitable materials should not only maintain their structural integrity for the products intended use, but also have the ability to disperse into small elements when flushed down a conventional toilet without causing blockage of the plumbing system. The materials also need to be stable and maintain integrity during storage, particularly in humid environments such as a bathroom or warehouse.

Absorbent articles typically include a body facing liquid permeable layer, an absorbent core and garment facing liquid impermeable barrier.

U.S. Pat. No. 3,683,919 purports to disclose a flushable sanitary napkin having a topsheet, absorbent core and backsheet. Prior to flushing, the topsheet and backsheet are torn apart and separated from the core. This can prove to be messy and inconvenient to the user.

Some materials may show the desired flushability but lacks stability, often losing strength and shape upon use. Other materials show stability but lack the ability to be safely flushed in normal sewage systems. For example, commercially available water soluble polymers, such as, polyethylene oxide (PEO), polyvinyl alcohol (PVOH), acrylamide polymers, acrylic acid-based polymers, and cellulose derivatives possess the desired characteristics for flushability but typically are unstable in use and storage. In particular, these polymers may get tacky in a moist environment including when handled by the user. Additionally, the disposable, flushable absorbent product should sufficiently breakdown when exposed to water and agitation so that it is transportable in the sewer system.

Other methods have been used to improve the flushability of absorbent articles by using specific materials that weaken when exposed to liquid or are biodegradable. See, for example, U.S. Pat. Nos. 6,359,063 ('063); 6,362,277 ('277); and US 5,026,589 ('589).

The '063 and '277 patents purport to disclose a flushable personal care article having a water-degradable polyolefin-containing film. In '063, film is made from grafting a monomer by free radical initiator onto a film made of a mixture of modified polyolefin and modified poly(ethylene oxide). In '277, poly(ethylene oxide) is unmodified. The components of the film are melt blended and extruded. The films lose at least 10 percent in two or more tensile properties after being immersed in water for 30 seconds. Such film does not provide quick breakdown in water, which makes it difficult to design articles for disposal in a sewer system.

The '589 patent purports to disclose a disposable absorbent structure having a water-permeable topsheet, which is a flexible sheet made from a dioxanone-based polymer. Additionally, the polymer topsheet may contain lactic acid and/or glycolic acid moieties. The dioxanone polymer used in the topsheet is designed to degrade by natural biological processes. A water insoluble topsheet and backsheet were disclosed, which prevents the product from being flushable.

An alternate to a polymer sheet includes paper. In general, paper is a material in which cellulose natural fibers are accumulated in sheet form and in which the fibers are linked by hydrogen bonds. When immersed in water, the hydrogen bonds are broken and the fiber is readily dispersed into fiber units. An example is toilet paper.

Paper is a type of material that exhibits too little wet strength to be useful in absorbent articles, some which must function in the presence of large amounts of moisture. In general, some paper tends to also be too stiff in their dry state to provide the comfort most consumers expect. To overcome these problems, paper may be coated or further treated to improve the physical properties. Examples of such treatments of cellulosic materials including wood pulp and cellulose fibers can be found in U.S. Pat. Nos. 3,844,987 ('987); 4,057,537 ('537); and 5,015,245 ('245).

The '987 patent purports to disclose a container made from biodegradable thermoplastic oxyalkanoyl polymers and naturally occurring biodegradable products such as hemp, linen, cotton, cardboard, wood pulp, disintegrated or shredded tree bark, peat moss, cotton seed hulls, disintegrated paper stock, shredded wood and other cellulosic products. In this disclosure, the term "biodegradable" is used for that type of degradability that is brought about by living organisms, usually microorganisms. The containers of the examples were typically buried in the ground and monitored for disintegration over a period of months.

The '537 patent purports to disclose copolymers made from optically active lactide and epsilon caprolactone that can be laminated into fibrous mats. The fibrous mats are made from natural and synthetic fibers such as cellulose derived from wood, cotton, linen, hemp, and the like. The copolymers of lactide and epsilon caprolactone are insoluble in water but upon constant contact with water are slowly degradable. '245 purports to disclose a paper backsheet treated with a polycationic latex material that enhances the wet strength of the paper. The latex material is water-insoluble and does not enhance the dry strength to the extent that the resulting article is unattractively stiff.

The dispersibility and solubility of water-soluble paper differs essentially from that of ordinary paper. When components, such as, carboxymethylcellulose ("CMC"), are added to cellulose fibers, the paper becomes water-soluble. The resulting paper has a water-soluble polymer that becomes a partially dissolved swollen body in water. Other polymers which can be utilized to make water-soluble paper includes polyvinyl pyrrolidone, polyethylene oxide (PEO), polyvinyl alcohol (PVOH), acrylamide polymers, acrylic acid-based polymers, and cellulose derivatives, e.g., hydroxypropylcellulose, and the like.

An example of a disposable absorbent article is disclosed in EP 1166803. This invention purports to provide a water decomposable absorbent article having a backsheet formed of a fibrous sheet containing water-dispersible fibers and water-insoluble CMC, where the CMC has a degree of etherification of between 0.3 and 0.6 and modified such that the hydrogens of at least 95% of carboxylic acids are substituted with metal. The CMC serves as a binder in the fibrous sheet in dry condition.

Ishikawa et al. in "Functional Paper in Specialty Paper" (Shikisai, Vol. 64, pp. 696-701 1991) discloses water-soluble paper in which CMC is compounded with paper in order to increase the characteristic of dispersion in water so that it can be dispersed and dissolved in water rapidly in the short time of 5 to 20 seconds.

What is needed is an absorbent article that retains its integrity when exposed to fluid but is disposable in that it is flushable. The absorbent article may dissolve upon exposure to water and may be biodegradable. Additionally, the absorbent article must be made from materials that are readily available and that are cost efficient.

### Summary of the Invention

A biodegradable, water dispersible absorbent article having a water insoluble, biodegradable cover; a tenderized absorbent core; a water insoluble, biodegradable film, and a water soluble outer shell wherein the water insoluble, biodegradable film is laminated to the absorbent core.

### Brief Description of the Drawings

FIG. 1 shows a perspective view of an embodiment of the present invention with a portion cut away; and
FIG. 2 shows an elevational view taken along line A-A of the embodiment of FIG. 1.

### Detailed Description of the Invention

"Dispersible" as used herein means the article or element exhibits visible changes after being placed in or exposed to water. This includes being placed in or flushed down a standard toilet. The changes may include any visible failures to the integrity of the article or element, such as holes, slits, shreds; breaking apart into smaller sections; dissolving; or a combination thereof.

"Biodegradable" as used herein means those materials that are capable of being broken down especially into innocuous products by the action of living things, such as, microorganisms.

"Water insoluble" as used herein relates to those materials that resist being dispersed or broken apart in water.

"Flushable" as used herein means discardable in a toilet, urinal, or other flushing device made for the purpose of receiving urine or other body exudates and transporting it through a sewage system, public or private.

"Agitation" as used herein means to move with irregular, rapid or violent action. In particular, agitation denotes the action of the water during flushing of any typical toilet or urinal. Normally, agitation possesses sufficient energy (of the water movement) to break apart the absorbent article or elements into smaller portions. These smaller portions are easily flushed and are acceptable for disposal in conventional water/sewer systems. Not all toilets have equivalent agitation; some newer toilets flush using gravity. Agitation may also include the force of a vortex formed when stirring water in a flask.

Absorbent articles that are useful in the present invention include any absorbent article that absorbs bodily fluids, excretions and discharges. Such absorbent articles include sanitary napkins, diapers, incontinence devices, breast pads, underarm shields, shoe liners, surgical dressings, adhesive bandages, tampons, and the like.

As used herein, the term "pantiliner" is intended to include all products conventionally used to absorb menstrual fluid or vaginal discharge, which are not tampons, whether referred to as sanitary napkins, panty shields, pantiliners or similar synonymous names.

In this invention, the absorbent article falls apart in water. As seen in the example, the absorbent article can be agitated by the swirling of water in a flask. Additionally, agitation may occur by the flushing action of a toilet. The absorbent article includes an absorbent structure that is a laminate made of a water soluble absorbent core and a water insoluble, biodegradable film. In one embodiment, the absorbent structure may be attached to a water soluble, outer shell. In another embodiment, a water insoluble, biodegradable cover is adhered to the water-soluble outer shell which encases the absorbent structure.

When the absorbent article is exposed to water, both absorbent structure and water soluble shell falls apart in water. This allows the article to separate into the two remaining intact components; that is the water insoluble, biodegradable cover and the water insoluble, biodegradable film. These components then further biodegrade in the waste stream.

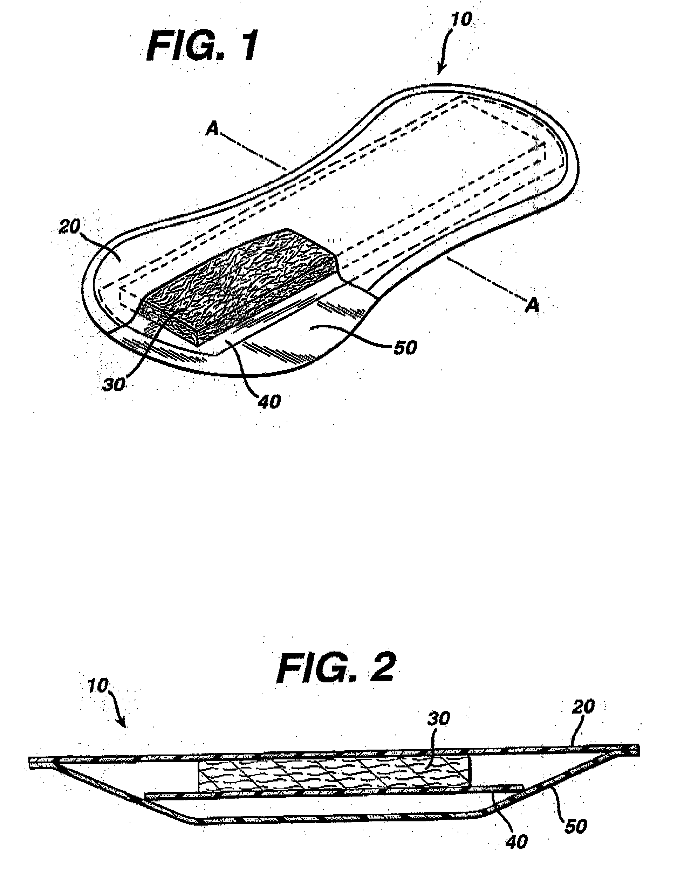

As seen in Figures 1 and 2, the absorbent article 10 is a pantiliner having a cover 20, an absorbent core 30, a film 40 and outer shell 50. While disposable article 10 is shown in Figure 1 as having a "peanut" shape or having concave longitudinal sides, disposable article 10 may have any shape such as rectangular, triangular, etc. Likewise, in the embodiment shown in Figure 1, the absorbent core 30 and film 40 each have a rectangular shape but may have any other shape.

### Cover

The absorbent article may include a water insoluble, biodegradable cover 20. The cover typically forms the body facing surface and overlays the absorbent structure and outer shell.

As known by those skilled in the art, the cover may be formed from any water insoluble biodegradable fluid pervious material that is comfortable against the skin and permits fluid to penetrate to the absorbent core, which retains the fluid. The cover should retain little or no fluid to provide a relatively dry surface next to the skin when in use. A variety of cover materials are known in the art, and any of these may be used.

Materials that can be used in making the cover include polycaprolactone (PCL), polylactic acid (PLA), pullulan and polyvinyl alcohol (PVOH). Also included are polyesters and natural and synthetic polymers, such as, water insoluble polysaccharides. Alternatively, if an extruded film is used, the cover may be apertured. In addition, such a film may be treated with a surfactant to increase hydrophilicity.

Generally, the cover is a single sheet or layer of material having a width sufficient to form the body-facing surface of the absorbent article. The cover may be longer and wider than the absorbent core.

It has been found that the cover having a density of at least about 9 gsm to about 20 gsm is useful in the present invention. More useful is a cover having a density of from about 9 to about 16,5 gsm. Included in these ranges are any densities that fall within the range. Additionally, it is found that the material used to form the cover should be hydrophilic, which can be by any methods known. to those skilled in the art, such as, surface treatment, surface spraying or incorporation of a surfactant into the mixture.

The cover material can be any water insoluble, biodegradable nonwoven material including but not limited to spunbond, spunlaced, hydroentangled, chemical and thermobonded material.

If the absorbent article includes a cover, it will typically overlay the entire absorbent structure. The exterior surface of the cover forms the body-facing surface of the absorbent article. The cover may be made from similar materials as the film.

Additionally, the absorbent article may include fluid acquisition layer (or transfer layer).

### Absorbent core

The absorbent core 30 may be any material currently used to absorb fluid or exudates. The material may or may not have a three-dimensional structure or it may be substantially flat.

Tenderizing is a process by which the material used to make the absorbent core is punctured by needles. Such processing aids in flexibility of the material, increases the surface area of the material, and makes the material more voluminous.

The absorbent core of an absorbent article of the present invention can be a fluffy batt cut from a relatively loose web of non-woven fibers having a relatively high absorptive capacity. While the absorbent core can have any shape or silhouette, it usually has an asymmetric configuration. The absorbent core may also be a fibrous batt having an integral densified layer. The densified layer has relatively higher wettability and liquid retentivity than the rest of the aforesaid batt and usually is formed by slightly moistening one surface of the batt and thereafter compressing the moistened surface. The absorbent core may also be formed from multiple layers, each having a different density such that the uppermost layer (closest to the body) is less dense than the outer (closest to the garment). Alternately, the absorbent core may be unitized with the cover.

The absorbent core of the present invention may contain any known absorbent materials including, but not limited to, absorbent fibers such as cellulose fibers, including, but not limited to wood pulp, regenerated cellulose fibers, and cotton fibers, rayon fibers and the like; superabsorbent fibers or particles; other naturally occurring absorbent materials, such as sphagnum or peat moss; and other synthetic absorbent materials, such as foams and the like. The absorbent core may also include one or more of the following: thermoplastic binder fibers, latex binder, perfumes, or odor-controlling compounds. The core may optionally be any substrate, e.g., tissue, woven, nonwoven, netted material, lace, or foam.

Additionally, the absorbent material can be an offline-formed, homogeneously mixed, air-laid layer, wet laid, roll good laminate or any other offline-formed absorbent composite.

In one embodiment, the absorbent core is made of absorbent material that is made from a layer of pulp. In another embodiment, superabsorbent polymer (SAP) is mixed with the pulp to form an absorbent composite. This composite may be condensed to form a dense, thin layer. One example of such a material is Novathin® available from Rayonier, Jesup, GA.

SAP are particles that are capable of absorbing many times, at least 10, more preferably 15, and still more preferably over 15, their weight in exudate, under a pressure of 0.5 psi. It should be noted that, in the context of the present invention, there is no restriction that the superabsorbent particles, actually be particulate. This expression is intended to cover superabsorbent fibers, and other superabsorbent materials, whatever their form and shape. These superabsorbent particles generally fall into three classes, namely starch graft copolymers, crosslinked carboxymethylcellulose derivates and modified hydrophilic polyacrylates. Examples of such absorbent polymers are hydrolyzed starch-acrylonitrile copolymer graft copblymer, a neutralized starch-acrylic acid graft copolymer, a saponified acrylic acid ester-vinyl acetate copolymer, a hydrolyzed acrylonitrile copolymer or acrylamide copolymer, a modified cross-linked polyvinyl alcohol, a neutralized self-cross-linking polyacrylic acid, a cross-linked polyacrylate salt, carboxylated cellulose, and a neutralized cross-linked isobutylene-maleic anhydride copolymer. In one embodiment of the invention, the superabsorbent particle is a cross-linked polyacrylate salt.

The superabsorbent particles are incorporated into the absorbent core in an amount no greater than about 60% on a weight per weight basis. Preferably, they are incorporated in an amount between about 40% and about 0.5% on a weight per weight basis. All specific amounts that are within the range are explicitly included in this disclosure. In the present context, 12% superabsorbent on "a weight per weight basis" is meant to mean 0.12 grams superabsorbent particles per 1 gram of all components comprising the absorbent core.

The absorbent core of the present invention may be constructed according to conventional techniques, e.g., by air-laying a mixture of wood pulp fibers and superabsorbent material. All such conventional techniques are within the scope of the present invention. In one embodiment, an absorbent layer is as described in U.S. Pat. No. 5,866,242 to Tan, which is herein incorporated by reference in its entirety.

The ratio of SAP to wood pulp may be varied over a wide range. If desired, a layer or multi-layer of dry laid type material can be used as the absorbent material to form the absorbent core. The absorbent material may be made of a superabsorbent polymer (SAP) of the type used in the art and wood pulp fibers having the desired density.

The absorbent core may also include additional materials such as odor control material, wetness indicator material, materials for administering or delivering medicaments, such as encapsulated medicaments, and materials for maintaining skin moisture, such as encapsulated moisturizers.

The absorbent core may be compressed or uncompressed, embossed or unembossed, or calendered. Embossing may be done by any conventional method and results in at least one boss and at least one valley disposed on the garment facing layer. The absorbent core may form a three-dimensional substrate.

### Film

The absorbent structure is formed from an absorbent core 30 and a film 40, which has been laminated together to form a unitary structure. The film may be any water insoluble, biodegradable polymers such as those used as the cover or alternately the film may be made from materials, such as, PCL, PLA, and PVOH. Other examples of water insoluble, biodegradable materials include polyortho esters, polyanhydrides, tyrosine-based polymers, polyphosphoesters, polyetheramides, polyesters including, but not limited to, poly(butylene succinate), poly(butylene succinate-co-adipate), poly(butylene succinate-co-carbonate), poly(butylene succinate-co-terephthalate), poly(ethylene succinate-co-adipate), poly(butylene adipate-co-terephthalate), poly(tetramethylene adipate-co-terephthalate), PCL, PLA, bacterial polyesters, such as, poly(hydroxyalkanoates), and PVOH. Natural polymers, such as, polysaccharides including starch and their blends, cellulose and their derivatives and biosynthetic polysaccharides may also be used.

The film may be adhered to the absorbent core by any method known to one of skill in the art. Such methods of adherence include mechanical methods, such as, heating, embossing, crimping, hooks, and the like, and chemical methods, such as, adhesives, including latex, solvents, and the like.

In one embodiment, an absorbent structure can be made of an absorbent core made of a wet laid pulp, available as Valucore® brand from Rayonier, Jesup, GA, which is laminated using hot melt adhesive to a 1 mil layer of PCL film. The PCL film is prepared from PCL resin available from Union Carbide, Midland, MI. The film is melt extruded at a temperature of from about 350° to about 420° F at 6 RPM.

It is useful to have the film extending beyond the lateral sides of the absorbent core to prevent leaking. One important aspect is to avoid including the film between the outer shell and the cover at the area where the lateral sides of the absorbent article are sealed.

### Outer shell

The outer shell 50 is water dispersible and may be made of any water soluble, fluid impermeable material. It may be formed from a laminate, composite or blend. It can also be a blend of water soluble and water insoluble materials or a composite of water soluble and water insoluble material. By having the water soluble component in the blend or composite, the water insoluble component will break apart upon exposure to water and agitation.

In one embodiment, a layer of water insoluble, biodegradable material is laminated or coated with a water soluble material. For example, an outer shell can be formed by coating a water-soluble PVOH resin to paper (cellulose fibers) on one side to form a laminated structure. The paper itself is not water soluble but can be biodegraded. PVOH is soluble upon exposure to water.

In another embodiment, a water insoluble material is blended with a water soluble material that acts as a binder. For example, a polyester material may be blended with a water soluble material such as PVOH. An example of this embodiment are blends of PCL and PVOH including about 25: about 75, about 50: about 50, and about 75: about 25 weight/weight blends that were melt extruded at about 350° to about 420°F (about 177 to about 216oC) at about 10 RPM into films.

Other examples of water insoluble, biodegradable materials include polyortho esters, polyanhydrides, tyrosine-based polymers, polyphosphoesters, polyetheramides, polyesters including, but not limited to, poly(butylene succinate), poly(butylene succinate-co-adipate), poly(butylene succinate-co-carbonate), poly(butylene succinate-co-terephthalate), poly(ethylene succinate-co-adipate), poly(butylene adipate-co-terephthalate), poly(tetramethylene adipate-co-terephthalate), PCL, PLA, bacterial polyesters, such as, poly(hydroxyalkanoates), and PVOH. Natural polymers, such as, polysaccharides including starch and their blends, cellulose and their derivatives and biosynthetic polysaccharides may also be used.

Different types of water dispersible papers may also be used. In one embodiment, the outer shell may have two layers: a layer of water soluble paper and a layer of PVOH. In another embodiment, the outer shell may have three layers: a layer of water-soluble paper coated on both sides by PVOH. Alternately, the paper pulp may be mixed with a water-soluble material. For example, the water-soluble paper components may be mixed with PVOH prior to forming the layer of water-soluble paper. The PVOH may act as a binder and be used to increase the strength and integrity of the paper layer. For example, Mishima Paper Co., LTD manufactures water soluble paper made from cellulose and CMC that can dissolve in water in about 10 to about 180 seconds. In particular, types A3015 and B3015 are extrusion laminated with PVOH and may be heat sealed to the absorbent structure. Other types of paper may be attached to the absorbent using construction adhesive or attached by other means. In another embodiment, A3015 paper (Mishima Paper Co., LTD) is laminated to the absorbent structure.

Any construction adhesive may be used to attach the layers into a single absorbent article. For example, in one embodiment, the body facing layer is attached to the outer shell with adhesive HL 1491 available from H.B Fuller and Company (St. Paul, MN). The adhesive may be applied by any method known to those skilled in the art.

Additionally, the absorbent core may be coated with a fluid permeable or impermeable material in a discrete pattern. Examples of this type of coating can be found in co-pending application entitled ABSORBENT ARTICLE INCLUDING IN SITU COVER, USSN 10/ , filed concurrently herewith, which is commonly owned and entirely incorporated herein by reference.

Secure attachment of absorbent article of the claimed invention to the garment contributes to maintaining the feeling of the user that the absorbent article and the garment are one in the same, i.e., permits the absorbent article to move with the underwear.

The absorbent article of the present invention may be applied to the crotch by placing the garment-facing surface against the inside surface of the crotch of the garment. Various methods of attaching absorbent articles may be used. For example, chemical means, e.g., positioning adhesive, and mechanical attachment means, e.g., clips, laces, ties, and interlocking devices, e.g., snaps, buttons, VELCRO (Velcro USA, Inc., Manchester, NH), zipper, and the like are examples of the various options available to the artisan.

Positioning adhesive may include pressure sensitive adhesive that is applied as strips, swirls, or waves, and the like. As used herein, the term pressure-sensitive adhesive refers to any releasable adhesive or releasable tenacious means. Suitable adhesive compositions, include, for example, water-based pressure-sensitive adhesives such as acrylate adhesives. Alternatively, the adhesive composition may include adhesives based on the following: emulsion or solvent-borne adhesives of natural or synthetic polyisoprene, styrene-butadiene, or polyacrylate, vinyl acetate copolymer or combinations thereof; hot melt adhesives based on suitable block copoylmers - suitable block copolymers for use in the invention include linear or radial co-polymer structures having the formula (A-B)x wherein block A is a polyvinylarene block, block B is a poly(monoalkenyl) block, x denotes the number of polymeric arms, and wherein x is an integer greater than or equal to one. Suitable block A polyvinylarenes include, but are not limited to Polystyrene, Polyalpha-methylstyrene, Polyvinyltoluene, and combinations thereof. Suitable Block B poly(monoalkenyl) blocks include, but are not limited to conjugated diene elastomers such as for example polybutadiene or polyisoprene or hydrogenated elastomers such as ethylene butylene or ethylene propylene or polyisobutylene, or combinations thereof. Commercial examples of these types of block copolymers include Kraton® elastomers from Kraton Ploymer Co, Vector® elastomers from Dexco, Solprene® elastomers from Enichem Elastomers and Stereon® from elastomers Firestone Tire & Rubber Co, hot melt adhesive based on olefin polymers and copolymers where in the olefin polymer is a terpolymer of ethylene and a co-monomers, such as vinyl acetate, acrylic acid, methacrylic acid, ethyl acrylate, methyl acrylate, n-butyl acrylate vinyl silane or maleic anhydride. Commercial examples of these types of polymers include Ateva® (polymers from AT plastics), Nucrel® (polymers from DuPont), Escor® (from Exxon Chemical).

Where adhesive is used, a release strip may be applied to protect the adhesive on the absorbent article prior to attaching the absorbent article to the crotch. The release strip can be formed. from any suitable sheet-like material which adheres with sufficient tenacity to the adhesive to remain in place prior to use but which can be readily removed when the absorbent article is to be used. Optionally, a coating may be applied to the release strip to improve the ease of removabilty of the release strip from the adhesive. Any coating capable of achieving this result may be used, e.g., silicone.

### Transfer Layer

The absorbent article of the present invention may include a transfer layer. The transfer layer may be made of any known material that will take up fluid and then distribute and release it to an adjacent absorbent core or layer for storage. Preferred transfer layers have a relatively open structure that allows for movement of fluid within the layer. Suitable materials for such transfer layers include any water dispersible material including fibrous webs, water dispersible resilient foams, and the like.

The transfer layer is able to accept fluid and allow passage of the fluid through its mass to be absorbed by an adjacent absorbent core. Thus, transfer layers that are made of hydrophobic, nonabsorbent fibers may be able to accept large volumes of fluid into inter-fiber void spaces while the fibers themselves do not absorb any significant quantities of fluid. Likewise, open-celled foam structures that are made from nonabsorbent materials may also absorb fluid into the cells of the foam. The walls of the cells, however, do not absorb any fluid. The cumulative spaces within the transfer layer, i.e., the inter-fiber void spaces in the fibrous transfer layer or the open cells in the foam transfer layer, function much like a container to hold fluid.

Transfer layers that are made from webs of mostly absorbent fibers absorb the fluid as it enters the structure and do not distribute it throughout the rest of the structure as efficiently as webs containing non-absorbent materials. Preferred transfer layer fibrous webs include nonabsorbent materials to provide void volume and to allow for free movement of fluid through the structure

The present invention may be used in making any shaped absorbent article. For example, pantiliners for use with thong-type underwear may be made using this invention. Additionally, the absorbent article may be tinted or colored, e.g., black, red, blue, and any color or mixture thereof, to offer discretion with the users undergarments.

The absorbent article may be packaged as unwrapped absorbent articles within a carton, box or bag. The consumer withdraws the ready-to-use article as needed. The absorbent article may also be individually packaged (each absorbent article encased within an overwrap).

Also contemplated herein, include asymmetrical and symmetrical absorbent articles having parallel longitudinal edges, dog bone- or peanut-shaped, and the like.

The absorbent article of the present invention may be used with conventional underwear or may be shaped to conform to thong garments. As used herein, the term thong includes, but is not limited to, thong underwear, thong swimming suit bottom, G-strings, Rio cut underwear, Rio cut swimming suit bottom, Brazilian cut underwear, Brazilian cut swimming suit bottom, and any other garment that exposes the buttocks, having a narrow strip of fabric or a cord that passes between the thighs supported by a waistband, a waist cord, belt or the garment itself.

From the foregoing description, one skilled in the art can ascertain the essential characteristics of this invention, and without departing from the spirit and scope thereof, can make various changes and modifications. Embodiments set forth by way of illustration are not intended as limitations on the variations possible in practicing the present invention.

### EXAMPLES

### Example 1

An absorbent article was made from the following materials:
Cover: 16.5 gsm spunbond PLA available from Shinwa;
Absorbent core: 175 gsm, 7% SAP, diamond embossed Novathin® from Rayonier;
Film: 0.8 mil ECO®-PLA from Dow-Cargill, LLC; and
Outer shell: A3015 water dispersible paper laminate available from Mishima Paper Co.

The absorbent core was laminated to the film by addition of construction adhesive (1491, Fuller). This absorbent structure was placed and adhered by construction adhesive to the outer shell. The cover was placed on top and the entire structure was sealed into a unitary structure by heat sealing the outer perimeter at various temperatures for predetermined periods of time.

### Example 2

Inventive samples were prepared using the materials from Example 1. The cover was sealed to the absorbent structure/outer shell under various temperatures:

| | |
|---|---|
| Inventive Sample 1 | 220oF for 4 seconds under 84 psi; |
| Inventive Sample 2 | 230oF for 4 seconds under 80 psi; |
| Inventive Sample 3 | 240oF for 4 seconds under 80 psi; |
| Inventive Sample 4 | 250oF for 4 seconds under 80 psi; |
| Inventive Sample 5 | 260oF for 4 seconds under 80 psi; and |
| Inventive Sample 6 | 270oF for 4 seconds under 80 psi. |

### Example 3

The inventive samples were immersed into a flask containing up to 1600 mL water at room temperature. The flask was secured on an orbital shaker platform until the rate indicated 200 rpm. A vortex was allowed to form in the flask. The inventive sample was placed into the center of the vortex. The amount of time it took for the inventive sample to break down from a unitary structure into the water insoluble cover and film was recorded. Each sample was measured in triplicate.

**Table 1**

| Sample Number | Dispersion Time (Seconds) |
|---|---|
| 1 | 6.4 |
| 2 | 9.4 |
| 3 | 10.0 |
| 4 | 6.3 |
| 5 | 7.3 |
| 6 | 7.9 |

The inventive samples of the present invention all had water dispersible times of 10 seconds or less.

## Claims

1. A biodegradable, water dispersible absorbent article comprising
an absorbent core,
a water insoluble, biodegradable film, and
a water dispersible outer shell,
wherein the water insoluble, biodegradable film is laminated to the absorbent core.

2. An absorbent article of claim 1 further comprising a water insoluble, biodegradable cover.

3. An absorbent article of claim 1, wherein the laminated film extends beyond an edge of the absorbent core.

4. An absorbent article of claim 1, wherein the water dispersible outer shell is made from a paper.

5. An absorbent article of claim 3, wherein the outer shell is a blendof paper and water soluble material.

6. An absorbent article of claim 3, wherein the outer shell is a blend of paper and a blend of water soluble materials and water insoluble materials.

7. An absorbent article of claim 3, wherein said outer shell is a paper coated with a blend of water soluble materials and water insoluble materials.

8. An absorbent article of claim 2, wherein the laminated film extends beyond an edge of the absorbent core.

9. An absorbent article of claim 2, wherein the water dispersible outer shell is made from a paper.

10. An absorbent article of claim 9, wherein the outer shell is blend of paper and water soluble material.

11. An absorbent article of claim 9, wherein the outer shell is a blend of paper and a blend of water soluble materials and water insoluble materials.

12. An absorbent article of claim 9, wherein said outer shell is a paper coated with a blend of water soluble materials and water insoluble materials.

13. An absorbent article of claim 3, wherein the absorbent core has first and second lateral sides and the film extends beyond the first and second lateral sides.

14. An absorbent article of claim 2, wherein the cover and outer shell are joined together forming an outer perimeter.

15. An absorbent article of claim 1, further comprising positioning adhesive on the outer shell for securing the absorbent article.

16. An absorbent article of claim 1, wherein the absorbent article disperses when placed in water in less than 5 minutes.

17. An absorbent article of claim 1, wherein the outer shell is blend of water soluble and water insoluble materials.
